# EUROPÄISCHE PATENTSCHRIFT

(11) **EP 0 957 692 B1**
(45) Veröffentlichungstag und Bekanntmachung des Hinweises auf die Patenterteilung: **06.08.2003**
(21) Anmeldenummer: 98904084.5
(22) Anmeldetag: 16.01.1998
(51) Int. Cl.: A23L 1/09, A61K 45/06

(54) **KOHLENHYDRATMISCHUNG**
CARBOHYDRATE MIXTURE
MELANGE D'HYDRATES DE CARBONE

(30) Priorität: 16.01.1997 DE 19701382
(43) Veröffentlichungstag der Anmeldung: 24.11.1999
(73) Patentinhaber: N.V. Nutricia, 2712 HM Zoetermeer (NL)
(72) Erfinder: STAHL, Bernd, D-61381 Friedrichsdorf (DE); SAWATZKI, Günther, D-35516 Münzenberg (DE)
(74) Vertreter: Köster, Hajo, Dr.
(86) Internationale Anmeldenummer: EP9800234
(87) Internationale Veröffentlichungsnummer: WO98031241

(56) Entgegenhaltungen:
- WO-A-92/10947
- WO-A-94/18986
- DE-A- 3 935 906

## Beschreibung

Die Erfindung betrifft eine Kohlenhydratmischung für diätetische, enterale und parenterale Nahrungen sowie Pharmazeutika und die Verwendung dieser Kohlenhydratmischung.

Kohlenhydrate stellen bekanntlich einen der wesentlichen Grundpfeiler der Ernährung dar. Daher werden die unterschiedlichsten Kohlenhydrate den verschiedensten Nahrungen, insbesondere "künstlich" hergestellten Nahrungen, und auch Pharmazeutika beigegeben. Die Aufgabe der Kohlenhydrate ist dabei primär nutritiver Art bzw. sie fungieren als Ballaststoff.

Die Kohlenhydrate bestehen aus Monosacchariden bzw. setzen sich aus diesen zusammen. Je nach Polymerisationsgrad werden die Kohlenhydrate als Oligosaccharide bzw. Polysaccharide oder Glycane bezeichnet. Im Rahmen der vorliegenden Unterlagen werden hier als Oligosaccharide Kohlenhydrate mit bis zu 6 Monosaccharid-Einheiten (und somit bis zum Hexasaccharid) verstanden. Kohlenhydrate mit 7 und mehr Monosacchariden (und somit ab Heptasaccharid) werden hier als Polysaccharide bezeichnet.

Aufgrund der Variabilität der die Kohlenhydrate aufbauenden Monomere, der Position der glycosidischen Bindung und der Anomerie der Kohlenhydrate stellen diese Kohlenhydrate und deren Konjugate eine extrem heterogene und umfangreiche Substanzklasse dar.

Kohlenhydrate haben nun die unterschiedlichsten biologischen Funktionen. In diesem Zusammenhang wird rein beispielhaft darauf verwiesen, daß Glycanstrukturen besonders bei Zell-Matrix-, Zell-Zell- und ähnlichen Erkennungs- und Adhäsionsprozessen eine wichtige Rolle spielen. Die Kohlenhydratstrukturen liegen sowohl als freie Oligosaccharide als auch in gebundener Form vor, beispielsweise in Glycoproteinen, Proteoglycanen und Glycolipiden. Die Adhäsion von Microorganismen an Glycostrukturen von Epithelien/Endothelien oder andere körpereigene Zellen wirkt sich unter anderem auch auf den Zellstoffwechsel des Wirtsorganismus aus. Die Liste der Funktionen, welche von Kohlenhydraten wahrgenommen werden, ließe sich nun beliebig verlängern. Die oben geschilderte Funktion der Glycanstrukturen stellt daher nur ein willkürlich gewähltes Beispiel dar.

So beschreibt die WO-A-92/10947 eine hypotonisches Getränk, das vor einer Operation oral verabreicht wird. Diese Getränk enthält ein wässrige Lösung einer Kohlenhydratmischung, welche jeweils mindestens ein Monosaccharid, ein Disaccharid und ein Polysaccharid in einer Menge von 8 bis 20 g pro 100 ml Lösung aufweist. Diese Kohlenhydratmischung soll den negativen Einfluss auf den Kohlenhydratmetabolismus bei einem Patienten nach einem chirurgischen Eingriff unterdrücken und die Abwehrkraft des Patienten bei chirurgisch bedingten Blutungen stärken.

Kohlenhydrate werden nun zunehmend in Nahrungen, "functional food" und Pharmazeutika unter dem Aspekt einer biologischen Wirksamkeit eingesetzt. Bisher wurden jedoch nur irgendwelche speziellen, über eine bestimmte Eigenschaft verfügenden Kohlenhydratspezies zur Anwendung gebracht.

Aufgabe der vorliegenden Erfindung ist es daher, eine Kohlenhydratmischung bereitzustellen, die diätetischen, enteralen und parenteralen Nahrungen sowie Pharmazeutika einverleibt werden kann und neben einem nutritiven Effekt auch über ein breites Wirkspektrum verfügt.

Gelöst wird diese Aufgabe durch die Lehre der Ansprüche.

Es wurde überraschend gefunden, daß die erfindungsgemäße Mischung von Monosacchariden, Oligosacchariden und Polysacchariden in ihrer biologischen Wirkung wesentlich potenter ist als die entsprechende Wirkung der Einzelkomponenten. Mit der erfindungsgemäßen Mischung können dabei folgende biologische Wirkungen erzielt werden:
- Verhinderung der Adhäsion von pathogenen Substanzen/Organismen wie Toxinen, Viren, Bakterien, Pilzen, transformierten Zellen und Parasiten
- Auflösung von Komplexen von Toxinen, Viren, Bakterien, Pilzen und anderen Pathogenen mit körpereigenen Zellen sowie deren Ausschleusung aus dem Körper
- Stabilisierung einer natürlichen Mikroflora
- Beschleunigung der Wundheilung (bei pharmazeutischen und enteralen Mischungen).

Damit eignet sich die erfindungsgemäße Mischung zur Prophylaxe und/oder Behandlung von Symptomen/Erkrankungen, die im Zusammenhang mit der Assoziation/Adhäsion der genannten Substanzen und Organismen an Epithelien oder andere körpereigenen Zellen stehen (wie Diarrhöe, Meningitis, Otitis, Gastritis und Influenza). Die erfindungsgemäße Mischung verfügt zudem aufgrund von Abbaureaktionen durch körpereigene Enzyme und nachfolgender Resorption der Produkte über eine nutritive Wirkung.

Während für die nutritiven und präbiotischen Effekte der erfindungsgemäßen Kohlenhydratmischung die Abbaurate, Kinetik und Resorption relevant sind, ist für die biologische Funktion überraschenderweise nicht nur die chemische Zusammensetzung sondern das Mischungsverhältnis von Monosacchariden/Oligosacchariden/Polysacchariden von Bedeutung.

Das Mischungsverhältnis von a = Monosaccharid, b = Oligosaccharid und c = Polysaccharid ist dabei erfindungsgemäß wie folgt: a = 1, b = 40 bis 1000, c = 1 bis 50.

Als Oligosaccharide werden dabei solche bis zum Hexasaccharid (z.B. Mono-, Di-, Tri-, Tetra-, Penta- und Hexasaccharid) verstanden.

Wenn im Rahmen der vorliegenden Unterlagen von einem Saccharid im Singular die Rede ist, dann kann es sich nicht nur um eine einzelne Spezies sondern auch um eine Mischung beliebiger Spezies handeln. Wenn zudem im Rahmen der vorliegenden Unterlagen von Bereichen die Rede ist, dann sind mit der Bereichsangabe zumindest alle ganzzahligen Zwischenwerte und auch von dem Bereich umfaßte engere Bereiche umfaßt und offenbart. Dies bedeutet beispielsweise für die Komponente c, die 1 bis 50 betragen kann, daß damit auch die dazwischenliegenden Werte, wie 2, 3, 4 ... 12, 13, 14 ... 25, 26, 27, ... 37, 38, 39, 40, 41 ... umfaßt sind. Analoges gilt für die Komponente b, so daß damit alle zwischen 40 bis 1000 liegenden, zumindest ganzzahligen Zwischenwerte (z.B. 41,42,43,44 .... bis 998,999) offenbart sind.

Die Mischung der Komponenten der erfindungsgemäßen Kohlenhydratmischung stellt somit ein wesentliches Merkmal der Erfindung dar. Das Mischungsverhältnis von Monosacchariden, Oligosacchariden und Polysacchariden beträgt dabei vorzugsweise 1:40:10 bis 1:1000:1 und insbesondere 1:80:20. Durch diese Angabe sind auch in diesem Fall alle zwischen den Bereichsgrenzen liegenden, insbesondere ganzzahligen Werte umfaßt und offenbart. Das Molekulargewicht der Polysaccharide kann dabei bis auf einige MDa und auf partikuläre Kohlenhydrate mit einer hohen Anzahl aktiver Gruppen ausgedehnt werden.

Ein weiteres wichtiges Merkmal der erfindungsgemäßen Mischung besteht darin, daß mindestens ca. 1 Gew.-% Fucose in dieser Mischung vorhanden ist (alle Mengenangaben beziehen sich im übrigen, sofern nichts anderes angegeben ist, auf das Gewicht). Die Fucose kann dabei in freier Form oder in gebundener Form (als fucosyliertes Oligosaccharid oder fucosyliertes Polysaccharid) vorliegen. Natürlich ist es auch möglich, daß die Fucose sowohl in freier Form als auch in gebundener Form vorliegt. Diese Fucose macht dabei vorzugsweise mindestens ca. 5 Gew.-% und insbesondere 5 bis 10 Gew.-% der erfindungsgemäßen Kohlenhydratmischung aus.

Nach einer weiterhin bevorzugten Ausführungsform enthält die erfindungsgemäße Kohlenhydratmischung zusätzlich ca. 1 Gew.-% Sialinsäure. Der Begriff Sialinsäure steht hier im Rahmen der vorliegenden Unterlagen stellvertretend für folgende Substanzen bzw. folgende Substanzen werden darunter subsummiert: N-Acetyl-Neuraminsäure, N-Glycolyl-Neuraminsäure und andere Neuraminsäuren. Alle diese Sialinsäuren können auch in O-acetylierter Form vorliegen. Die Sialinsäure kann dabei frei vorliegen oder an ein Oligosaccharid und/oder an ein Polysaccharid gebunden sein. Natürlich sind auch beliebige Mischungen möglich. Die Sialinsäure macht dabei vorzugsweise 1 bis 5 Gew.-% aus und liegt insbesondere als Sialyllactose und/oder Disialyllactose bzw. Disialyllacto-N-tetraose vor.

Zur Herstellung der erfindungsgemäßen Kohlenhydratmischung kann man alle bisher bekannten und insbesondere für die Herstellung von Nahrungen bzw. Nahrungsmitteln eingesetzten Kohlenhydrate und Kohlenhydratmischungen einsetzen. Auch ist es möglich, bereits durch technische Modifikation veränderte Rohstoffe zur Anwendung zu bringen. Die Herstellung der erfindungsgemäßen Mischung kann dann durch einfaches Mischen der entsprechend ausgewählten Monosaccharide, Oligosaccharide und Polysaccharide in dem gewünschten Mischungsverhältnis erfolgen.

Als Rohstoffe können somit sowohl freie Kohlenhydrate wie Speicherkohlenhydrate (Stärke, Fructane) als auch Gerüstkohlenhydrate wie Zellulosen, Hemizellulosen und Chitine eingesetzt werden. Darüberhinaus können Glycokonjugate wie Glycolipide, Glycoproteine, Proteoglycane etc. eingesetzt werden. Auch ist es möglich, eine enzymatische Modifikation mit Hydrolasen (beispielsweise Glycosidasen, Transglycosidasen und Lipasen), Transferasen (beispielsweise Fucosyl-Transferasen und Sialyltransferasen), Isomerasen (beispielsweise Aldolasen und Ketolasen), Oxidoreduktasen (beispielsweise Oxidasen) und Reduktasen (Glucosedehydrogenase)), Lyasen (beispielsweise Polysaccharidlyase) und Ligasen der Rohstoffe und Produkte durchzuführen. Ferner ist es möglich, eine technische Modifikation der Rohstoffe und Produkte vorzunehmen, nämlich durch Druck (beispielsweise Extrusion), Temperatur (beispielsweise Karamelisierung), organische Synthesen, organische Modifizierung (beispielsweise Carboxymethylierung und Peracetylierung), saure und/oder basische Hydrolyse und Fraktionierung (beispielsweise nach Größe und/oder physikochemischen Parametern wie Ladung und Hydrophobizität).

Die erfindungsgemäße Kohlenhydratmischung setzt sich dabei im wesentlichen aus den nachstehend aufgeführten Monosacchariden und den daraus aufgebauten Oligosacchariden sowie Polysacchariden zusammen:
N-Acetylneuraminsäure, N-Glycolylneuraminsäure und/oder deren O-acetylierte Formen, D-Glucose, D-Fructose, D-Galactose, D-Mannose, L-Fucose, D-N-Acetylglucosamin, D-N-Acetylgalactosamin, D-Xylose, L-Rhamnose, D-Arabinose, D-Allose, D-Talose, L-ldose, D-Ribose und Monosaccharide mit Carboxylgruppen wie D-Galacturonsäure.

Diese Monomere und die darauf aufgebauten höheren Einheiten können außerdem durch -OSO₃H- und/oder OPO₃H-Gruppen modifiziert sein.

Die Fucosylierung bzw. Sialisierung der Kohlenhydrate kann dabei auf übliche Weise erfolgen.

Wie bereits oben dargelegt, kann das Mischungsverhältnis a:b:c bis zu 1:1000:1 betragen. Dies ist insbesondere dann der Fall, wenn nur wenig freie Fucose und/oder freie Sialinsäure vorhanden ist.

Das Verhältnis der gesamten neutralen Kohlenhydrate zu den gesamten sauren Kohlenhydraten (beispielsweise NeuAc und/oder Kohlenhydraten mit OSO₃H- und/oder OPO₃H-Gruppen sollte vorzugsweise 100:1 bis 1:1 und insbesondere bevorzugt 10:1 betragen.

Die in der erfindungsgemäßen Mischung zum Einsatz gebrachten Monosaccharide und Oligosaccharide weisen zwar eine niedrige Affinität (Anbindung oder Komplexierung) für bestimmte Rezeptoren auf, können aber aufgrund der erhöhten Diffusion und Flexibilität überraschenderweise bereits bestehende Komplexe von Substanzen und Organismen (Toxinen/Viren, Bakterien und Zellen) mit den Zielstrukturen (beispielsweise Epitheloberflächen) und den damit assoziierten Erkennungsmolekülen (d.h. Rezeptoren) auflösen.

Die in der erfindungsgemäßen Mischung vorhandenen Polysaccharide weisen viele Epithope (Bindestellen) auf und besitzen somit eine um einige Größenordnung höhere Affinität und damit eine starke Bindung (verglichen mit den kleinen Molekülen) zu den entsprechenden Rezeptoren. Zusätzlich wird durch Moleküle mit derartigen polyvalenten Bindestellen eine Quervernetzung vieler Erkennungsmoleküle erzielt. Diese Polysaccharide können somit Substanzen und Organismen, beispielsweise Bakterien, gegebenenfalls aber auch deren Rezeptoren und gesamte (Epithel)-Oberflächen stabil maskieren. Eine Adhäsion der Substanzen und Organismen, beispielsweise Bakterien, an Zelloberflächen und den gesamten Epithelien wird somit verhindert. Zudem wird die Ausschleusung bzw. Vernichtung der Substanzen und Organismen aus dem Körper begünstigt.

Die Fucose - und Sialinsäure-Einheiten beeinflussen die biologische Aktivität von Oligo- und Polysacchariden.

Die erfindungsgemäßen Kohlenhydratmischungen werden vorzugsweise bei Säuglingen in einer Menge von mindestens 100mg/kg Körpergewicht/Tag, insbesondere von 500 mg/kg/Tag (bei Erkrankung doppelte Dosis), eingesetzt. Bei Erwachsenen werden die erfindungsgemäßen Kohlenhydratmischungen vorzugsweise in einer Menge von mindestens 200 mg/kg Körpergewicht/Tag, insbesondere von 1 g/kg/Tag (bei Erkrankung doppelte Dosis) verabreicht.

Nachstehend sind verschiedene, bevorzugte Ausführungsformen darstellende Kohlenhydratmischungen beschrieben. Die Angaben beziehen sich dabei auf Gew.-%, sofern nichts anderes angegeben ist.

### Beispiel 1

| Zusammensetzung | |
|---|---|
| Bestandteil | Gew.-% |
| Fucose | 0,5 |
| Glucose | 0,5 |
| Sialyllactosen | 1 |
| Fucosyllactosen | 0,5 |
| Fucosylierte Oligosaccharide wie | |
| Lactofucopentaosen | 4 |
| Sialisierte Oligosaccharide wie | |
| Disialolacto-N-Tetraose | 1 |
| Maltodextrin | 72,5 |
| Stärke | 20 |

### Beispiel 2

| Zusammensetzung | |
|---|---|
| Bestandteil | Gew.-% |
| Fucose | 0,5 |
| Glucose | 0,5 |
| Sialyllactosen | 1 |
| Fucosyllactosen | 0,5 |
| Fucosylierte Oligosaccharide wie | |
| Difucosyl-Lactose | 4 |
| Sialisierte Oligosaccharide wie | |
| Sialolacto-N-Hexaosen | 1 |
| Inulin | 72,5 |
| Stärke | 20 |

### Beispiel 3

| Zusammensetzung | |
|---|---|
| Bestandteil | Gew.-% |
| Fucose | 1,5 |
| Glucose | 0,5 |
| Sialyllactosen | 1 |
| Fucosyllactosen | 0,5 |
| Fucosylierte Oligosaccharide wie Difucosyl-Lacto-N-Tetraose | 3 |
| Sialisierte Oligosaccharide wie Sialolacto-N-Hexaosen | 1 |
| Oligosaccharide wie Mannane und Galactane | 10 |
| Inulin | 62,5 |
| Mikrokristalline Cellulose | 20 |

### Beispiel 4

| Zusammensetzung | |
|---|---|
| Bestandteil | Gew.-% |
| Fucose | 1,5 |
| Glucose | 0,5 |
| Sialyllactosen | 1 |
| Fucosyllactosen | 0,5 |
| Fucosylierte Oligosaccharide wie Lactofucopentaosen | 3 |
| Sialisierte Oligosaccharide wie Disialolacto-N-Tetraose | 1 |
| Galactooligosaccharide | 10 |
| Inulin | 62,5 |
| Mikrokristalline Cellulose | 20 |

### Beispiel 5

| Zusammensetzung | |
|---|---|
| Bestandteil | Gew.-% |
| Fucose | 0,1 |
| Glucose | 0,5 |
| Sialyllactose | 0,5 |
| Fucosyllactosen | 4,9 |
| Disialyllacto-N-tetraose | 2,0 |
| Maltodextrin | 70 |
| Stärke | 22 |

### Beispiel 6

| Zusammensetzung | |
|---|---|
| Bestandteil | Gew.-% |
| Fucose | 0,1 |
| Glucose | 0,5 |
| Sialyllactose | 1 |
| Fucosyllactosen | 0,9 |
| Galacto-Oligosaccharide | 20,5 |
| Inulin | 5 |
| Stärke | 72 |

## Patentansprüche

1. Kohlenhydratmischung für diätetische, enterale und parenterale Nahrungen sowie Pharmazeutika,
**dadurch gekennzeichnet,**
**dass** sie aus a = Monosaccharid(en), b = Oligosaccharid(en) und c = Polysaccharid(en) mit einem Mischungsverhältnis a : b : c von, bezogen auf das Gewicht, :
a = 1
b = 40 bis 1000 und
c = 1 bis 50,
aufgebaut ist und
mindestens 1 Gew.-% Fucose in freier und/oder in an ein Oligosaccharid und/oder ein Polysaccharid gebundener Form enthält.

2. Kohlenhydratmischung nach Anspruch 1,
**dadurch gekennzeichnet,**
**dass** das Mischungsverhältnis a:b:c ca. 1:80:20 beträgt.

3. Kohlenhydratmischung nach Anspruch 1 oder 2,
**dadurch gekennzeichnet,**
**dass** sie mindestens 5 Gew.-% und insbesondere 5 bis 10 Gew.-% Fucose enthält.

4. Kohlenhydratmischung nach einem der vorhergehenden Ansprüche,
**dadurch gekennzeichnet,**
**dass** die Fucose an die Oligosaccharide und Polysaccharide wie folgt gebunden ist : α 1-2, α 1-3, α 1-4, α 1-6.

5. Kohlenhydratmischung nach einem der vorhergehenden Ansprüche,
**dadurch gekennzeichnet,**
**dass** sie mindestens 1 Gew.-% Sialinsäure(n) in freier und/oder in an ein Oligosaccharid und/oder ein Polysaccharid gebundener Form enthält.

6. Kohlenhydratmischung nach Anspruch 5,
**dadurch gekennzeichnet,**
**dass** sie 1 bis 5 Gew.-% Sialinsäure(n), insbesondere in gebundener Form als Sialyllactose und/oder Disialyllactose bzw. Disialyllacto-N-tetraose, enthält.

7. Kohlenhydratmischung nach Anspruch 5 oder 6,
**dadurch gekennzeichnet,**
**dass** die Sialinsäure(n) an die Oligosaccharide und Polysaccharide wie folgt gebunden ist (sind): α 2-3, α 2-6, α 2-8.

8. Kohlenhydratmischung nach einem der vorhergehenden Ansprüche,
**dadurch gekennzeichnet,**
**dass** die Monosaccharide im wesentlichen aus den folgenden Monomeren bestehen bzw. die Oligosaccharide und Polysaccharide im wesentlichen aus den folgenden Monomeren zusammengesetzt sind, die durch -OSO3H- und/oder -OPO3H-Gruppen modifiziert sein können:
N-Acetylneuraminsäure, N-Glycolylneuraminsäure und/oder deren O-acetylierte Formen, D-Glucose, D-Fructose, D-Galactose, D-Mannose, L-Fucose, D-N-Acetylglucosamin, D-N-Acetylgalactosamin, D-Xylose, L-Rhamnose, D-Arabinose, D-Allose, D-Talose, L-Idose, D-Ribose und Monosaccharide mit Carboxylgruppen wie D-Galacturonsäure.

9. Kohlenhydratmischung nach einem der vorhergehenden Ansprüche,
**dadurch gekennzeichnet,**
**dass** es sich bei den Monosacchariden, Oligosacchariden und Polysacchariden um per se bekannte und für die Herstellung von Kohlenhydratmischungen üblicherweise eingesetzte Saccharide handelt, die fucosyliert und gegebenenfalls sialysiert sind oder wurden.

10. Kohlenhydratmischung nach einem der vorhergehenden Ansprüche
**dadurch gekennzeichnet,**
**dass** sie eine diätetische, enterale oder parenterale Nahrung oder ein Pharmazeutikum darstellt oder einer derartigen Nahrung oder Pharmazeutikum einverleibt ist.

11. Verwendung einer Kohlenhydratmischung nach einem der Ansprüche 1 bis 9 zur Herstellung einer diätetischen, enteralen oder parenteralen Nahrung oder eines Pharmazeutikums zur Prophylaxe und/oder Behandlung von Symptomen und Erkrankungen, die im Zusammenhang mit der Assoziation bzw. Adhäsion von pathogenen Substanzen und/oder Organismen an Epithelien oder andere körpereigene Zellen stehen, insbesondere bei Säuglingen.

## Claims

1. Carbohydrate mixture for dietetic, enteral and parenteral foods and also pharmaceuticals,
**characterised in that**,
it is made up of a = monosaccharide(s), b = oligosaccharide(s) and c = polysaccharide(s) with a mixing ratio a : b : c, based on weight,
a = 1
b = 40 to 1000 and
c = 1 to 50, and
contains at least 1 wt % fucose in free form and/or in a form bound to an oligosaccharide and/or a polysaccharide.

2. Carbohydrate mixture according to claim 1,
**characterised in that**,
the mixing ratio a : b : c is ca. 1 : 80 : 20.

3. Carbohydrate mixture according to claim 1 or 2,
**characterised in that**,
it contains at least 5 wt % and in particular 5 to 10 wt % fucose.

4. Carbohydrate mixture according to one of the foregoing claims,
**characterised in that**,
the fucose is bound to the oligosaccharides and polysaccharides as follows:
α 1-2, α 1-3, α 1-4, α 1-6.

5. Carbohydrate mixture according to one of the foregoing claims,
**characterised in that**,
it contains at least 1 wt % sialic acid(s) in free form and/or a form bound to an oligosaccharide and/or a polysaccharide.

6. Carbohydrate mixture according to claim 5,
**characterised in that**,
it contains 1 to 5 wt % sialic acid(s), in particular in bound form as sialyllactose and/or disialyllactose or disialyllacto-N-tetraose.

7. Carbohydrate mixture according to claim 5 or 6,
**characterised in that**,
the sialic acid(s) is (are) bound to the oligosaccharides as follows:
α 2-3, α 2-6, α 2-8.

8. Carbohydrate mixture according to one of the foregoing claims,
**characterised in that**,
the monosaccharides essentially consist of the following monomers or the oligosaccharides and polysaccharides are essentially composed of the following monomers, which can be modified by_-OSO₃H and/or -OPO₃H groups:
N-acetylneuraminic acid, N-glycolylneuraminic acid and/or O-acetylated forms thereof, D-glucose, D-fructose, D-galactose, D-mannose, L-fucose, D-N-acetylglucosamine, D-N-acetylgalactosamine, D-xylose, L-rhamnose, D-arabinose, D-allose, D-talose, L-idose, D-ribose and monosaccarides with carboxyl groups such as D-galacturonic acid.

9. Carbohydrate mixture according to one of the foregoing claims,
**characterised in that**,
the monosaccharides, oligosaccharides and polysaccharides are saccharides well-known per se and normally used for the production of carbohydrate mixtures, which can be or have been fucosylated and optionally sialised.

10. Carbohydrate mixture according to one of the foregoing claims,
**characterised in that**,
it is a dietetic, enteral and parenteral food or a pharmaceutical or is incorporated in such a food or pharmaceutical.

11. Use of a carbohydrate mixture according to one of claims 1 to 9 for the production of a dietetic, enteral and parenteral food or a pharmaceutical for the prophylaxis and/or treatment of symptoms and diseases, which are connected with the association or adhesion, respectively, of pathogenic substances and/or organisms to epithelia or other endogenous cells, in particular in the case of infants.

## Revendications

1. Mélange de sucres pour des aliments diététiques, par voie digestive et parentérale ainsi que pour des produits pharmaceutiques,
**caractérisé**
**en ce qu'**il est composé de a = monosaccharide(s), de b = oligosaccharide(s) et de c = polysaccharide(s), avec un rapport de mélange a:b:c, par rapport au poids, de
a = 1
b = 40 à 1 000, et
c = 1 à 50,
et
**en ce qu'**il contient du fucose, à raison d'au moins 1% en poids, sous forme libre et/ou liée à un oligosaccharide et/ou à un polysaccharide.

2. Mélange de sucres selon la revendication 1,
**caractérisé**
**en ce que** le rapport de mélange a:b:c est d'environ 1:80:20.

3. Mélange de sucres selon la revendication 1 ou 2,
**caractérisé**
**en ce qu'**il contient du fucose à raison d'au moins 5% en poids et en particulier à raison de 5% à 10% en poids.

4. Mélange de sucres selon l'une quelconque des revendications précédentes,
**caractérisé**
**en ce que** le fucose est lié aux oligosaccharides et aux polysaccharides comme suit : α 1-2, α 1-3, α 1-4, α 1-6.

5. Mélange de sucres selon l'une quelconque des revendications précédentes,
**caractérisé**
**en ce qu'**il contient un acide sialique ou des acides sialiques, à raison d'au moins 1% en poids, sous forme libre et/ou liée à un oligosaccharide et/ou à un polysaccharide.

6. Mélange de sucres selon la revendication 5,
**caractérisé**
**en ce qu'**il contient un acide sialique ou des acides sialiques, à raison de 1% à 5% en poids, en particulier liés sous forme de sialyllactose et/ou de disialyllactose ou de disialyllacto-N-tétraose.

7. Mélange de sucres selon la revendication 5 ou 6,
**caractérisé**
**en ce que** l'acide sialique ou les acides sialiques sont liés aux oligosaccharides et aux polysaccharides comme suit : α 2-3, α 2-6, α 2-8.

8. Mélange de sucres selon l'une quelconque des revendications précédentes,
**caractérisé**
**en ce que** les monosaccharides sont essentiellement composés des monomères suivants, ou que les oligosaccharides et les polysaccharides sont essentiellement composés des monomères suivants qui peuvent être modifiés par des groupes -OSO₃H-et/ou -OPO₃H- :
l'acide N-acétylneuraminique, l'acide N-glycolylneuraminique et/ou leurs formes O-acétylées, le D-glucose, le D-fructose, le D-galactose, le D-mannose, le L-fucose, la D-N-acétylglucosamine, la D-N-acétylgalactosamine, le D-xylose, le L-rhamnose, le D-arabinose, le D-allose, le D-talose, le L-idose, le D-ribose et les monosaccharides possédant des groupes carboxyle tels que l'acide D-galacturonique.

9. Mélange de sucres selon l'une quelconque des revendications précédentes,
**caractérisé**
**en ce que** les monosaccharides, les oligosaccharides et les polysaccharides sont des saccharides qui sont connus en soi et habituellement mis en oeuvre pour la préparation de mélanges de sucres, saccharides que l'on soumet ou que l'on a soumis à une fucosylation et, éventuellement, à une sialysation.

10. Mélange de sucres selon l'une quelconque des revendications précédentes,
**caractérisé**
**en ce qu'**il représente un aliment diététique, par voie digestive ou parentérale ou un produit pharmaceutique, ou bien en ce qu'il est incorporé dans un aliment ou un produit pharmaceutique de ce type.

11. Mise en oeuvre des mélanges de sucres selon l'une quelconque des revendications 1 à 9, pour la préparation d'un aliment diététique, par voie digestive ou parentérale ou bien d'un produit pharmaceutique destiné à la prophylaxie et/ou au traitement de symptômes et de maladies qui sont liés à l'association ou à l'adhérence d'organismes et/ou de substances pathogènes à des épithéliums ou à d'autres cellules du corps, en particulier chez les nourrissons.
